# EUROPEAN PATENT SPECIFICATION

(11) **EP 1 320 379 B1**
(45) Date of publication and mention of the grant of the patent: **28.04.2010**
(21) Application number: 01965468.0
(22) Date of filing: 13.09.2001
(51) Int. Cl.: A61K 39/015, A61K 39/00, A61K 39/04, A61P 31/06, A61P 31/04, A61P 31/12, A61P 33/06

(54) **USE OF REPLICATION-DEFICIENT POXVIRUS VECTOR TO BOOST CD4+ T CELL IMMUNE RESPONSE TO ANTIGEN**
VERWENDUNG EINES REPLIKATIONDEFIZIENTEN POCKENVIRUSVEKTORS ZUR ERHÖHUNG DER CD4+ T-ZELL-IMMUNANTWORT GEGEN ANTIGEN
UTILISATION D'UN VECTEUR DE POXVIRUS DEFICIENT POUR LA REPLICATION POUR STIMULERLA REPONSE IMMUNITAIRE DES LYMPHOCYTES T CD4+ CONTRE L'ANTIGENE

(30) Priority: 21.09.2000 GB 0023203
(43) Date of publication of application: 25.06.2003
(73) Proprietor: Oxxon Therapeutics Limited, Oxford, Oxfordshire OX14 4SH (GB)
(72) Inventor: HILL, Adrian, V. S., Oxford OX3 9DL (GB); McSHANE, Helen, Oxford OX2 7RF (GB); GILBERT, Sarah, Horspath, Oxford O X33 1SD (GB); REECE, William, Oxford OX4 2NY (GB); SCHNEIDER, Joerg, Oxford OX3 8BT (GB)
(74) Representative: Goodfellow, Hugh Robin
(86) International application number: PCT/GB2001/004116
(87) International publication number: WO 2002/024224

(56) References cited:
- WO-A-98/56919
- RAMSHAW I A ET AL: "The prime-boost strategy: exciting prospects for improved vaccination" IMMUNOLOGY TODAY, ELSEVIER PUBLICATIONS, CAMBRIDGE, GB, vol. 21, no. 4, April 2000 (2000-04), pages 163-165, XP004194963 ISSN: 0167-5699
- PLEBANSKI MAGDALENA ET AL: "Protection from Plasmodium berghei infection by priming and boosting T cells to a single class I-restricted epitope with recombinant carriers suitable for human use." EUROPEAN JOURNAL OF IMMUNOLOGY, vol. 28, no. 12, December 1998 (1998-12), pages 4345-4355, XP002192944 ISSN: 0014-2980
- JOHNSON R P ET AL: "INDUCTION OF A MAJOR HISTOCOMPATIBILITY COMPLEX CLASS I-RESTRICED CYTOTOXIC T-LYMPHOCYTE RESPONSE TO A HIGHLY CONSERVED REGION OF HUMAN IMMUNODEFICIENCY VIRUS TYPE 1 (HIV-1) GP120 IN SERONEGATIVE HUMANS IMMUNIZED WITH A CANDIDATE HIV-1 VACCINE" JOURNAL OF VIROLOGY, THE AMERICAN SOCIETY FOR MICROBIOLOGY, US, vol. 68, no. 5, May 1994 (1994-05), pages 3145-3153, XP001027055 ISSN: 0022-538X
- SCHNEIDER J ET AL: "ENHANCED IMMUNOGENICITY FOR CD8+ T CELL INDUCTION AND COMPLETE PROTECTIVE EFFICACY OF MALARIA DNA VACCINATION BY BOOSTING WITH MODIEFIED VACCINIA VIRUS ANKARA" NATURE MEDICINE, NATURE PUBLISHING, CO, US, vol. 4, no. 4, April 1998 (1998-04), pages 397-402, XP000739989 ISSN: 1078-8956
- DENIS O ET AL: "Vaccination with plasmid DNA encoding Mycobacterial Antigen 85A stimulates a CD4+ and CD8+ T-cell epitopic repertoire broader than that stimulated by Mycobacterium tuberculosis H37Rv infection" INFECTION AND IMMUNITY, AMERICAN SOCIETY FOR MICROBIOLOGY. WASHINGTON, US, vol. 66, no. 4, April 1998 (1998-04), pages 1527-1533, XP002173898 ISSN: 0019-9567
- MCSHANE HELEN ET AL: "Enhanced immunogenicity of CD4+ T-cell responses and protective efficacy of a DNA-modified vaccinia virus Ankara prime-boost vaccination regimen for murine tuberculosis." INFECTION AND IMMUNITY, vol. 69, no. 2, February 2001 (2001-02), pages 681-686, XP002192945 ISSN: 0019-9567
- A. LALVANI ET AL.: "Human cytolytic and interferon gamma-secreting CD8+ T lymphocytes specific for Mycobacterium tuberculosis" PNAS USA, vol. 95, 1998, pages 270-275,
- A. LALVANI & A.V.S. HILL: "Cytotoxic T-lymphocytes against malaria and tuberculosis: from natural immunity to vaccine design" vol. 95, 1998, pages 531-538,
- S.M. SMITH ET AL.: "Characterization of human Mycobacterium bovis bacille Calmette-Guerin-reactive CD8+ T cells" vol. 67, no. 10, 1999, pages 5223-5230,
- A.A. PATHAN ET AL.: "High frequencies of circulating IFN-gamma-secreting CD8 cytotoxic T cells specific for a novel MHC class I-restricted Mycobacterium tuberculosis epitope in M. tuberculosis-infected subjects without disease" EUROPEAN JOURNAL OF IMMUNOLOGY, vol. 30, no. 9, September 2000 (2000-09), pages 2713-2721,

## Description

The present invention relates to a method of inducing a CD4+ T cell response against a target antigen using a composition comprising a source of CD4+ T cell epitopes.

### BACKGROUND

Although subunit vaccines that elicit strong antibody responses have been available some years, it has been more difficult to design vaccines that stimulate the cellular arm of the immune system to produce strong protective T lymphocyte responses.

Much attention has been focused on inducing CD8+ T cells that may be cytolytic and have been found to protect against some viral infections. In contrast CD4+ T cells have, until recently, usually been regarded as helper T cells that play a role in helping other immunocytes to generate protection, for example by amplifying antibody responses.

However, recent evidence has shown that CD4+ T cells can also be effector cells that play a more direct role in protection. For example in the case of tuberculosis, malaria and H. pylori infection there is evidence for a protective role of CD4 T cells that can secrete the cytokine, gamma-interferon.

There is thus a need for the development of vaccines which are capable of stimulating an effective CD4+ T cell response. Poxviruses are known to be good inducers of CD8 T cell responses because of their intracytoplasmic expression. However, they are generally believed to be poor at generating CD4 MHC class II restricted T cells (see for example Haslett et al. Journal of Infectious Diseases 181:1264-72 (2000), page 1268)

### Tuberculosis

More than one hundred years after Koch's discovery of the causative organism, tuberculosis remains a major global public health problem. There are estimated to be 8-10 million new cases per annum and the annual mortality is approximately 3 million. The variability in protective efficacy of the currently available vaccine, *Mycobacterium bovis* bacillus Calmette-Guérin (BCG) [1], and the advent of multi-drug resistant strains of tuberculosis, means that there is an urgent need for a better vaccine.

*M. tuberculosis* is an intracellular pathogen and the predominant immune response involves the cellular arm of the immune system. There is strong evidence from animal and human studies that CD4+ T cells are necessary for the development of protective immunity [2,3]. There is also increasing evidence that CD8+ T cells may play a role [4,5].

DNA vaccines are inducers of cellular immune responses, inducing both CD4+ and CD8+ T cells, and therefore represent a promising delivery system for a tuberculosis vaccine. A number of studies assessing the protective efficacy of DNA vaccines encoding a variety of antigens from *M tuberculosis* have shown partial protection against challenge that is equivalent to the protection conferred by BCG [6,7]. However, none of the vaccine candidates tested so far has been found to be consistently superior to BCG. Although DNA vaccines are good at eliciting both CD4+ and CD8+ T cells, the frequency of response cells they produce may need to be significantly increased in order to confer protection against challenge.

There is thus a need for alternative and improved vaccines capable of inducing a CD4+ T cell response, optionally in conjunction with a CD8+ T cell response for protection against diseases such as tuberculosis.

WO98/56919 described a method of generating a CD8+ T cell immune response against malaria and other diseases, by administering a priming composition containing a CD8+ T cell epitope followed by a boosting composition comprising a non-replicating or replication-impaired pox virus vector carrying at least one CD8+ T cell epitope which was the same as the epitope in the priming composition.

Ramshaw & Ramsay (Immunology Today 21(4):163-165 2000) described "prime-boost" regimens for generating immune responses against viruses including HIV and influenza, involving priming with a DNA vaccine and boosting with a viral vector.

Johnson et al. (J. Virology 68(5):3145-3153 1994) described generation of CD4+ and CD8+ T cell immune responses against HIV using a primary immunisation with a recombinant vaccinia virus expressing gp160 and then a booster immunisation with recombinant gp160, and reported the identification of CD8+ CTL epitopes from conserved regions of gp120.

### SUMMARY OF THE INVENTION

The present inventors have now shown that replication-defective pox viruses are capable of inducing effector CD4+ T cells that are protective. Using a gamma-interferon ELISPOT assays with cell subset depletion they have proved that these CD4+ effector T cells are well induced in both mice and humans after immunisation. The use of heterologous prime-boost regimes with replication-impaired poxviruses induces strong CD4 T cell responses.

Thus the present invention provides use of:
(a) a first composition comprising a source of one or more CD4+ T cell epitopes of a tuberculosis antigen; and
(b) a second composition comprising a source of one or more CD4+ T cell epitopes of the tuberculosis antigen, including at least one CD4+ T cell epitope which is the same as a CD4+ T cell epitope of the first composition,
wherein the source of T cell epitopes in the first composition is not a poxvirus vector,
wherein the source of CD4+ epitopes for the second composition is a non-replicating or replication impaired recombinant poxvirus vector;
for the manufacture of a medicament for inducing a T_{H}1-type CD4+ T cell response against the tuberculosis antigen, wherein the doses of the first and second compositions may be administered in either order.

If the vector also provides a source of CD8+ T-cell epitopes, then the method of the present invention may induce a combined CD4+/CD8+ T-cell response.

If the source of epitopes in (a) is a viral vector, the viral vector in (b) is derived from a different virus.

The first and second compositions used in the method of the present invention may conveniently be provided in the form of a kit.

In addition to heterologous prime-boost regimes, the present inventors have shown that replication-defective pox viruses are capable of acting as boosting agents for pre-existing CD4+ T cell responses. Thus the present invention also provides use of a source of one or more CD4+ T cell epitopes of a tuberculosis antigen, wherein the source is a recombinant non-replicating or replication-impaired pox virus vector, in the manufacture of a medicament for boosting a primed T_{H}1-type CD4+ T cell immune response.

The capacity of recombinant replication-impaired poxvirus vectors to induce such functional CD4+ T cell responses, both when used alone and in prime-boost combinations, in both animals and in man, has widespread utility both for prophylactic and for therapeutic vaccination against tuberculosis.

### BRIEF DESCRIPTION OF THE FIGURES

Figure 1: A graph to show the efficacy of various immunisation regimes after 8 weeks. Data represent the mean and standard error of 7-15 mice/group.
Figure 2: A graph showing the results of a ⁵¹Cr Release assay performed on the splenocytes from mice in the DDDM group
Figure 3: A graph comparing heterologous and homologous regime's protection to challenge. Mean CFU counts/organ were taken at 8 weeks. *, p < 0.05; **, p < 0.01 when compared to the naive control group.

### DETAILED DESCRIPTION OF INVENTION

### CD4 +/CD8+ immune responses

T cells fall into two major groups which are distinguishable by their expression of either the CD4 or CD8 co-receptor molecules. CD8-expressing T cells are also known as cytotoxic T cells by virtue of their capacity to kill infected cells. CD4-expressing T cells, on the other hand, have been implicated in mainly "helping" or "inducing" immune responses.

The nature of a T cell immune response can be characterised by virtue of the expression of cell surface markers on the cells. T cells in general can be detected by the present of TCR, CD3, CD2, CD28,CD5 or CD7 (human only). CD4+ T cells and CD8+ T cells can be distinguished by their co-receptor expression (for example, by using anti-CD4 or anti-CD8 monoclonal antibodies, as is described in the Examples).

Since CD4+ T cells recognise antigens when presented by MHC class II molecules, and CD8+ recognise antigens when presented by MHC class I molecules, CD4+ and CD8+ T cells can also be distinguished on the basis of the antigen presenting cells with which they will react.

Within a particular target antigen, there may be one or more CD4+ T cell epitopes and one or more CD8+ T cell epitopes. If the particular epitope has already been characterised, this can be used to distinguish between the two subtypes of T cell, for example on the basis of specific stimulation of the T cell subset which recognises the particular epitope.

CD4+ T cells can also be subdivided on the basis of their cytokine secretion profile. The T_{H}1 subset (sometimes known as "inflammatory CD4 T cells") characteristically secretes IL-2 and IFNγ and mediates several functions associated with cytotoxicity and local inflammatory reactions. T_{H}1 cells are capable of activating macrophages leading to cell mediated immunity. The T_{H}2 subset (sometimes known as "helper CD4 T cells") characteristically secretes Il-4, IL-5, IL-6 and IL-10, and is thought to have a role in stimulating B cells to proliferate and produce antibodies (humoral immunity).

T_{H}1 and T_{H}2 cells also have characteristic expression of effector molecules. T_{H}1 cells expressing membrane-bound TNF and T_{H}2 cells expressing CD40 ligand which binds to CD40 on the B cell.

The CD4+ T cell response induced by the method of the present invention is a TH1-type response. Preferably the induced CD4+ T cells are capable of secreting IFNγ.

The induction of a CD4+ or CD8+ immune response will cause an increase in the number of the relevant T cell type. This may be detected by monitoring the number of cells, or a shift in the overall cell population to reflect an increasing proportion of CD4+ or CD8+ T cells). The number of cells of a particular type may be monitored directly (for example by staining using an anti-CD4/CD8 antibody, and then analysing by fluorescence activated cell scanning (FACScan)) or indirectly by monitoring the production of, for example a characteristic cytokine. In the Examples the presence of CD4+ T cells is monitored on the basis of their capacity to secrete IFNγ, in response to a specific peptide, using an ELISPOT assay. CD4 and CD8 T cell responses are readily distinguished in ELISPOT assays by specific depletion of one or other T cell subset using appropriate antibodies. CD4 and CD8 T cell responses are also readily distinguished by FACS (fluorescence activated cell sorter) analysis.

### CD4+/CD8+ T cell epitopes

A T cell epitope is a short peptide derivable from a protein antigen. Antigen presenting cells can internalise antigen and process it into short fragments which are capable of binding MHC molecules. The specificity of peptide binding to the MHC depends on specific interactions between the peptide and the peptide-binding groove of the particular MHC molecule.

Peptides which bind to MHC class I molecules (and are recognised by CDB+ T cells) are usually between 6 and 12, more usually between 8 and 10 amino acids in length. The amino-terminal amine group of the peptide makes contact with an invariant site at one end of the peptide groove, and the carboxylate group at the carboxy terminus binds to an invariant site at the other end of the groove. The peptide lies in an extended confirmation along the groove with further contacts between main-chain atoms and conserved amino acid side chains that line the groove. Variations in peptide length are accomodated by a kinking in the peptide backbone, often at proline or glycine residues.

Peptides which bind to MHC class II molecules are usually at least 10 amino acids, more usually at least 13 amino acids in length, and can be much longer. These peptides lie in an extended confirmation along the MHC II peptide-binding groove which is open at both ends. The peptide is held in place mainly by main-chain atom contacts with conserved residues that line the peptide-binding groove.

For a given antigen, CD4+ and CD8+ epitopes may be characterised by a number of methods known in the art. When peptides are purified from cells, their bound peptides co-purify with them. The peptides can then by eluted from the MHC molecules by denaturing the complex in acid, releasing the bound peptide, which can be purified (for example by HPLC) and perhaps sequenced.

Peptide binding to many MHC class I and II molecules has been analysed by elution of bound peptides and by X-ray crystallography. From the sequence of a target antigen, it is possible to predict, to a degree, where the Class I and Class II peptides may lie. This is particularly possible for MHC class I peptides, because peptides that bind to a given allelic variant of an MHC class I molecule have the same or very similar amino acid residues at two or three specific positions along the peptide sequence, known as anchor residues.

Also, it is possible to elucidate CD4+ and CD8+ epitopes using overlapping peptide libraries which span the length of the target antigen. By testing the capacity of such a library to stimulate CD4+ or CD8+ T cells, one can determine the which peptides are capable of acting as T cell epitopes. In the examples, a peptide library for two antigens from *M tuberculosis* are analysed using an ELISPOT assay.

### Sources of T cell epitopes

The CD4+ and optionally CD8+ T cell epitopes either present in, or encoded by the compositions, may be provided in a variety of different forms; such as a recombinant string of one or two or more epitopes, or in the context of the native target antigen, or a combination of both of these. CD4+ and CD8+ T cell epitopes have been identified and can be found in the literature, for many different diseases. It is possible to design epitope strings to generate a CD4+ and/or CD8+T cell response against any chosen antigen that contains such epitopes. Advantageously, the epitopes in a string of multiple epitopes are linked together without intervening sequences so that unnecessary nucleic acid and/or amino acid material is avoided. In addition to the CD4+ T cell epitopes from the target antigen, it may be preferable to include one or more other epitopes recognised by T helper cells, to augment the immune response generated by the epitope string. Particularly suitable T helper cell epitopes are ones which are active in individuals of different HLA types, for example T helper epitopes from tetanus (against which most individuals will already be primed).

Preferably, the source of CD4+ (and optionally CD8+) T cell epitopes in the first composition in the method according to the invention is a non-viral vector or a non-replicating or replication-impaired viral vector, although replicating viral vectors may be used.

The source of T cell epitopes in the first composition is not a poxvirus vector, so that there is minimal cross-reactivity between the first and second compositions.

Alternative preferred viral vectors for use in the first composition according to the invention include a variety of different viruses, genetically disabled so as to be non-replicating or replication-impaired. Such viruses include for example non-replicating adenoviruses such as El deletion mutants. Genetic disabling of viruses to produce non-replicating or replication-impaired vectors is well known.

Other suitable viral vectors for use in the first composition are vectors based on herpes virus and Venezuelan equine encephalitis virus (VEE). Suitable bacterial vectors for the first composition include recombinant BCG and recombinant Salmonella and Salmonella transformed with plasmid DNA (Darji A et al 1997 Cell 91: 765-775).

Alternative suitable non-viral vectors for use in the priming composition include lipid-tailed peptides known as lipopeptides, peptides fused to carrier proteins such as KLH either as fusion proteins or by chemical linkage, whole antigens with adjuvant, and other similar systems.

In one preferred embodiment of the invention, the source of T cell epitopes in the first composition is a nucleic acid, which may be DNA or RNA, in particular a recombinant DNA plasmid. The DNA or RNA may be packaged, for example in a lysosome, or it may be in free form.

In another preferred embodiment of the invention, the source of T cell epitopes in the first composition is a peptide, polypeptide, protein, polyprotein or particle comprising two or more CD4+ T cell epitopes, present in a recombinant string of CD4+ T cell epitopes or in a target antigen. Polyproteins include two or more proteins which may be the same, or preferably different, linked together. Preferably the epitopes in or encoded by the first or second composition are provided in a sequence which does not occur naturally as the expressed product of a gene in the parental organism from which the target antigen may be derived.

Preferably, the source of T cell epitopes in the second composition is a vaccinia virus vector such as MVA or NYVAC. Most preferred is the vaccinia strain modified virus ankara (MVA) or a strain derived therefrom (more detail on MVA is provided below). Alternatives to vaccinia vectors include avipox vectors such as fowl pox or canarypox vectors. Particularly suitable as an avipox vector is a strain of canarypox known as ALVAC (commercially available as Kanapox), and strains derived therefrom.

### Poxvirus vectors

In the "second" composition used in the method of the present invention the source of the CD4+ (and optionally CD8+) epitopes is a non-replicating or replication impaired recombinant poxvirus vector.

The term "non-replicating" or "replication-impaired" as used herein means not capable of replication to any significant extent in the majority of normal mammalian cells or normal human cells. Viruses which are non-replicating or replication-impaired may have become so naturally (i.e. they may be isolated as such from nature) or artificially e.g. by breeding *in vitro* or by genetic manipulation, for example deletion of a gene which is critical for replication. There will generally be one or a few cell types in which the viruses can be grown, such as CEF cells for MVA.

Replication of a virus is generally measured in two ways: 1) DNA synthesis and 2) viral titre. More precisely, the term "nonreplicating or replication-impaired" as used herein and as it applies to poxviruses means viruses which satisfy either or both of the following criteria:
1) exhibit a 1 log (10 fold) reduction in DNA synthesis compared to the Copenhagen strain of vaccinia virus in MRC-5 cells (a human cell line);
2) exhibit a 2 log reduction in viral titre in HELA cells (a human cell line) compared to the Copenhagen strain of vaccinia virus.

Examples of poxviruses which fall within this definition are MVA, NYVAC and avipox viruses, while a virus which falls outside the definition is the attenuated vaccinia strain M7.

Modified vaccinia virus Ankara (MVA) is a strain of vaccinia virus which does not replicate in most cell types, including normal human tissues. MVA was derived by serial passage > 500 times in chick embryo fibroblasts (CEF) of material derived from a pox lesion on a horse in Ankara, Turkey (Mayr et al. Infection (1975) 33: 6-14.). It was shown to be replication-impaired yet able to induce protective immunity against veterinary poxvirus infections. MVA was used as a human vaccine in the final stages of the smallpox eradication campaign, being administered by intracutaneous, subcutaneous and intramuscular routes to > 120,000 subjects in southern Germany. No significant side effects were recorded, despite the deliberate targeting of vaccination to high risk groups such as those with eczema (Mayr et al. Bakteriol B. (1978)167: 375- 90).

The safety of MVA reflects the avirulence of the virus in animal models, including irradiated mice and following intracranial administration to neonatal mice. The non-replication of MVA has been correlated with the production of proliferative white plaques on chick chorioallantoic membrane, abortive infection of non-avian cells, and the presence of six genomic deletions totalling approximately 30 kb. The avirulence of MVA has been ascribed partially to deletions affecting host range genes K1 L and C7L, although limited viral replication still occurs on human TK-143 cells and African Green Monkey CV-1 cells. Restoration of the K1 L gene only partially restores MVA host range. The host range restriction appears to occur during viral particle maturation, with only immature virions being observed in human HeLa cells on electron microscopy (Sutter et al. 1992). The late block in viral replication does not prevent efficient expression of recombinant genes in MVA.

Poxviruses have evolved strategies for evasion of the host immune response that include the production of secreted proteins that function as soluble receptors for tumour necrosis factor, IL-I p, interferon (IFN)-oc/ andIFN-y, which normally have sequence similarity to the extracellular domain of cellular cytokine receptors (such as chemokine rcecptors).

These viral receptors generally inhibit or subvert an appropriate host immune response, and their presence is associated with increased pathogenicity. The Il-I p receptor is an exception: its presence diminishes the host febrile response and enhances host survival in the face of infection. MVA lacks functional cytokine receptors for interferon y, interferon ap, Tumour Necrosis Factor and CC chemokines, but it does possess the potentially beneficial IL-1 receptor. MVA is the only known strain of vaccinia to possess this cytokine receptor profile, which theoretically renders it safer and more immunogenicthan other poxviruses. Another replication impaired and safe strain of vaccinia known as NYVAC is fully described in Tartaglia et al.(Virology 1992, 188: 217-232).

Poxvirus genomes can carry a large amount of heterologous genetic information. Other requirements for viral vectors for use in vaccines include good immunogenicity and safety. In one embodiment the poxvirus vector may be a fowlpox vector, or derivative thereof.

It will be evident that vaccinia virus strains derived from MVA, or independently developed strains having the features of MVA which make MVA particularly suitable for use in a vaccine, will also be suitable for use in the invention.

MVA containing an inserted string of epitopes has been previously described in WO 98/56919.

### Vaccination strategies

The present inventors have shown that replication-defective pox viruses are capable of inducing effector CD4 T cells (optionally with CD8+ T cells) when used in heterologous prime-boost regimes.

Surprisingly, strong responses were obtained using a heterologous immunisation regime with the first and second compositions in either order. A slightly stronger was response was observed when the second composition was administered after the first, rather than the reverse order. At least one dose of the first composition may be administered, followed by at least one dose of the second composition.

A plurality of doses of the first composition may be administered followed by at least one dose of the second composition.

A particularly effective immunisation protocol has been found to be the administration of three sequential doses of the first composition, followed by one dose of the second composition.

The timing of the individual doses will depend on the individual (see "Administration" below) but will commonly be in the region of one to six weeks apart, usually about three weeks apart.

### Target antigens

The target antigen in the present invention is a tuberculosis antigen. It may be an antigen which is recognised by the immune system after infection with the disease. Alternatively the antigen may be normally "invisible" to the immune system such that the method induces a non-physiological T cell response. This may be helpful in diseases where the immune response triggered by the disease is not effective (for example does not succeed in clearing the infection) since it may open up another line of attack.

In a preferred embodiment of the invention, the antigen is derivable from M *tuberculosis.* For example, the antigen may be ESAT6 or MPT63.

The compositions of the present invention may comprise T cell epitopes from more than one antigen (see above under "epitopes"). For example, the composition may comprise one or more T cell epitopes from two or more antigens associated with the same disease. The two or more antigens may be derivable from the same pathogenic organism.

Alternatively, the composition may comprise epitopes from a variety of sources. For example, the ME-TRAP insert described in the examples comprises T cell epitopes from *P. falciparum,* tetanus toxoid, *M tuberculosis* and *M. bovis.*

### Target Diseases

The present invention will be useful in the prevention of tuberculosis.

The present invention is particularly useful in vaccination strategies to protect against tuberculosis.

The compositions described herein may be employed as therapeutic or prophylactic vaccines.

### Kits

The first and second compositions used in the method of the invention may conveniently be provided in the form of a "combined preparation" or kit. The first and second compositions may be packaged together or individually for separate sale. The first and second compositions may be used simultaneously, separately or sequentially for inducing a CD4+ T cell response against a target antigen.

The kit may comprise other components for mixing with one or both of the compositions before administration (such as diluents, carriers, adjuvants etc.- see below).

The kit may also comprise written instructions concerning the vaccination protocol.

### Pharmaceutical compositions/Vaccines

A medicament for boosting a primed CD4+ T cell response may be in the form of a pharmaceutical composition.

The pharmaceutical composition may also comprise, for example, a pharmaceutically acceptable carrier, diluent, excipient or adjuvant. The choice of pharmaceutical carrier, excipient or diluent can be selected with regard to the intended route of administration and standard pharmaceutical practice.

In particular, a composition comprising a DNA plasmid vector may comprise granulocyte macrophage-colony stimulating factor (GM-CSF), or a plasmid encoding it, to act as an adjuvant; beneficial effects are seen using GM-CSF in polypeptide form. Adjuvants such as QS21 or SBAS2 (Stoute J A et al. 1997 N Engl J Medicine 226: 86-91) may be used with proteins, peptides or nucleic acids to enhance the induction of T cell responses.

In the pharmaceutical compositions of the present invention, the composition may also be admixed with any suitable binder(s), lubricant(s), suspending agent(s), coating agent(s), or solubilising agent(s).

The pharmaceutical composition could be for veterinary (i.e. animal) usage or for human usage.

### Administration

In general, a therapeutically effective daily oral or intravenous dose of the compositions of the present invention is likely to range from 0.01 to 50 mg/kg body weight of the subject to be treated, preferably 0.1 to 20 mg/kg. The compositions of the present invention may also be administered by intravenous infusion, at a dose which is likely to range from 0.001-10 mg/kg/hr.

Tablets or capsules of the agents may be administered singly or two or more at a time, as appropriate. It is also possible to administer the compositions of the present invention in sustained release formulations.

Typically, the physician will determine the actual dosage which will be most suitable for an individual patient and it will vary with the age, weight and response of the particular patient. The above dosages are exemplary of the average case. There can, of course, be individual instances where higher or lower dosage ranges are merited, and such are within the scope of this invention.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intracavernosally, intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

For some applications, preferably the compositions are administered orally in the form of tablets containing excipients such as starch or lactose, or in capsules or ovules either alone or in admixture with excipients, or in the form of elixirs, solutions or suspensions containing flavouring or colouring agents.

For parenteral administration, the compositions are best used in the form of a sterile aqueous solution which may contain other substances, for example enough salts or monosaccharides to make the solution isotonic with blood.

For buccal or sublingual administration the compositions may be administered in the form of tablets or lozenges which can be formulated in a conventional manner.

For oral, parenteral, buccal and sublingual administration to subjects (such as patients), the daily dosage level of the agents of the present invention may typically be from 10 to 500 mg (in single or divided doses). Thus, and by way of example, tablets or capsules may contain from 5 to 100 mg of active agent for administration singly, or two or more at a time, as appropriate. As indicated above, the physician will determine the actual dosage which will be most suitable for an individual patient and it will vary with the age, weight and response of the particular patient. It is to be noted that whilst the above-mentioned dosages are exemplary of the average case there can, of course, be individual instances where higher or lower dosage ranges are merited and such dose ranges are within the scope of this invention.

In some applications, generally, in humans, oral administration of the agents of the present invention is the preferred route, being the most convenient and can in some cases avoid disadvantages associated with other routes of administration - such as those associated with intracavernosal (i.c.) administration. In circumstances where the recipient suffers from a swallowing disorder or from impairment of drug absorption after oral administration, the drug may be administered parenterally, e.g. sublingually or buccally.

For veterinary use, the composition of the present invention is typically administered as a suitably acceptable formulation in accordance with normal veterinary practice and the veterinary surgeon will determine the dosing regimen and route of administration which will be most appropriate for a particular animal. However, as with human treatment, it may be possible to administer the composition alone for veterinary treatments.

### EXAMPLES

### EXAMPLE 1: Immunogenicity and protective efficacy against tuberculosis

### Example 1A - ESAT6 and MPT63 Antigens

Since secreted antigens that are released from live mycobacteria are thought to be important in the generation of protective immunity, the present inventors selected two secreted antigens from *M. tuberculosis* for inclusion in the vaccines. The first, ESAT6 (early secreted antigenic target 6), is relatively specific for *M*. *tuberculosis,* and importantly, is not present in *M. bovis* BCG [10]*.* ESAT 6 is a key antigenic target early in murine infection [11] and is a human CTL target [5]. The second antigen, MPT63 (mycobacterial protein tuberculosis 63), is present in some strains *of M. bovis* BCG [12]. A polyprotein DNA construct and recombinant MVA virus containing both antigens were generated and we assessed the immunogenicity of these constructs, individually and in combination. The most immunogenic vaccine combinations were then assessed in murine challenge experiments with *M tuberculosis.*

### Construction of plasmid DNA and recombinant MVA tuberculosis vaccines

A single coding sequence containing the TPA leader sequence, ESAT6 and MPT63 genes and the Pk antibody epitope (TEMPk) was constructed and ligated into the plasmid vector pSG2, creating the DNA vaccine pSG2.TEMPk. Expression of the fusion protein was shown to be in the cytoplasm.

The recombinant MVA was purified from a transfection of wild type MVA and a vaccinia shuttle vector containing the sequence TEMPk.

### DNA and MVA vaccines both induce peptide-specific IFNγ producing CD4+ T cells.

C57B1/6 mice were immunised with DNA(i.m), MVA(i.d.) or a combination of the two. Using overlapping peptides which span the length of both antigens and an IFN-γ ELISPOT assay, we identified responses to several peptides in the splenocytes of immunised mice. Responses were seen to two peptides within ESAT 6 (E1 and E2) and four peptides within MPT63 (M3,15,27 and 28) (Table 1). To assess the phenotype of the cells responding to these peptides, CD4+ and CD8+ cells were depleted using magnetic beads. The responses to all six peptides were fully abrogated when CD4+ T cells were depleted, and unaffected when CD8+ T cells were depleted (Table 1). Assays were performed both ex-vivo (peptides E1 and E2) and after culturing the cells with peptide for 5-7 days (all peptides, see methods).

**Table 1. Peptides showing T cell epitopes identified in ESAT 6 and MPT63**

| **Antigen** | **Peptide** | **Sequence** | **CD4** | **CD8** |
|---|---|---|---|---|
| ESAT 6 | 1 | MTEQQ WNFAG IEAAA | + | - |
| | 2 | WNFAG IEAAA SAIQG | + | - |
| MPT63 | 3 | VAVVA MAAIA TFAAP | + | - |
| | 15 | VAGQV WEATA TVNAI | + | - |
| | 27 | GKIYF DVTGP SPTIN | + | - |
| | 28 | DVTGP SPTIV AMNGM | + | - |

### Homologous boosting of DNA and MVA induced responses

The highest frequency of IFN-γ secreting T cells (SFC) was to the first ESAT 6 peptide, E1 (Table 2). A single dose of DNA failed to generate any detectable responses, but when repeated twice, or three times at two weekly intervals, consistent responses were seen. After three immunisations, the number of SFC was more than double that seen after two immunisations; the mean response to E1 after two doses was 30 SFC per 10⁶ splenocytes, and after three doses was 75 SFC. A single immunisation with MVA generated responses with a mean frequency of spot forming cells to E1 of 20 per million, which were modestly improved following a second dose of MVA (mean frequency of SFC to E1 = 30). Thus gamma-interferon secreting CD4 T cell responses are induced to an encoded CD4 T cell epitope by immunisation with this replication-impaired poxvirus.

**Table 2. Summary of peptide specific T cell responses to the two constructs.**

| Condition | SFC to individual peptides | | | | | |
|---|---|---|---|---|---|---|
| | E1 | E2 | M3 | M15 | M27 | M28 |
| DNA x 1 | - | - | - | - | - | - |
| DNA x 2 | 30 | 5 | - | 5 | 5 | 5 |
| DNA x 3 | 75 | 50 | - | 15 | 40 | 45 |
| MVA x 1 | 20 | 10 | 5 | - | 5 | 5 |
| MVA x 2 | 30 | 10 | 5 | - | 10 | 10 |
| DNA/MVA | 130 | 26 | 12 | 17 | 5 | 10 |
| MVA/DNA | 130 | 70 | 10 | 10 | 10 | 5 |
| DNAx3/MVA | 360 | 250 | 25 | 100 | 30 | 50 |

### a) Numbers represent mean of 3-10 mice per 10⁶ splenocytes. Standard error is < 20%.

### Heterologous prime-boost regimes increased the magnitude of the observed CD4 T lymphocyte responses

Having detected these responses using the vaccines individually, the role of heterologous prime-boost regimes, i.e. using either the DNA or MVA construct to prime the response and the second construct to boost two weeks later, was assessed. Heterologous boosting, either DM or MD, produced stronger responses than homologous boosting of either DD or MM (Table 2). The mean response to peptide E1 was increased by more than 4-fold to 130 SFC and, surprisingly - in view of the finding on induced CD8 T cell responses [8]-, this occurred regardless of which order the two vaccines were given. The response to peptide E2 was slightly stronger when MVA was followed by DNA rather than the reverse order. The responses to peptides M3 and M15 were stronger with a heterologous boost, regardless of the order in which the vaccines were given, whilst the responses to peptides M27 and M28 were weak and not boosted. The strongest response was seen when DNA was given three times and then boosted with MVA once (DDDM). In this case the mean response to E1 was increased almost 5-fold from 75SFC to 360SFC. The responses to the other peptides were also higher after DDDM compared with DM.

In further studies using plasmid DNAs expressing the M. tuberculosis antigen, Ag85A and a recombinant MVA expressing the same antigen the induction of CD4 T cells to a CD4 T cell epitope was observed in Balb/c mice. Depletion studies using antibody coated beads confirmed that the response to the P15 peptide in this antigen was CD4-dependent. In the same experiment CD8 T cells were also induced to the P15 CTL epitope in Ag85 by both DNA immunisation and by recombinant MVA immunisation. Stronger response to both the CD4 and CD8 epitope were observed after prime-boost immunisation, priming with DNA and boosting with MVA. Thus both recombinant MVA immunisation and heterologous prime-boost immunisation can generate CD4 and CD8 gamma-interferon-secreting T cell responses to epitopes in the same antigen.

### Challenge experiments

Heterologous prime-boost regimes generated the highest levels of IFN-γ secreting CD4+ T cells, and therefore the protective efficacy of these regimes was assessed in challenge experiments using the ESAT-MPT63 constructs. The first challenge experiment compared the protective efficacy of DNA prime / MVA boost (DM), with MVA prime / DNA boost (MD) supplemented in each case with a second MVA boost. The second challenge experiment assessed the protection conferred by three sequential immunisations with DNA followed by a single MVA immunisation (DDDM). In both experiments, BCG was used as a positive control. The immunogenicity of each vaccine regime was assessed in 2-3 mice before the remainder of the group were challenged. In the first experiment, the immune responses were not as strong as previously seen (average response to E1 25 SFC). Therefore in this case a second dose of MVA was administered to both groups prior to challenge. In the second challenge experiment, the average response to the dominant peptide, E1, was 225 SFC. The challenge was conducted two weeks after the last subunit vaccine immunisation, shortly after the T cell response had reached a plateau (unpublished data).

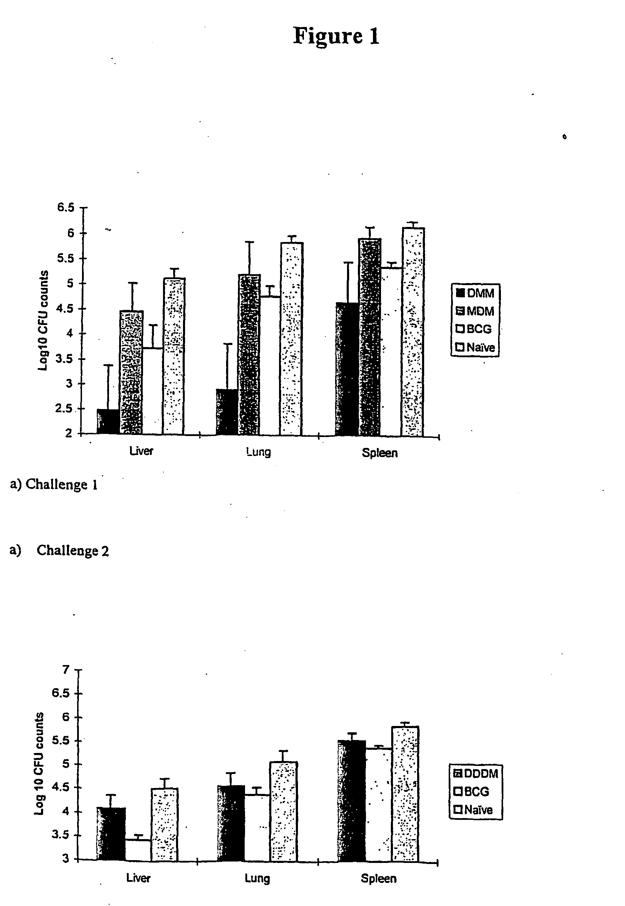

To assess the efficacy of the immunisation regimes at 8 weeks, organs from all mice remaining at 8 weeks were harvested, and CFU counts determined. In both challenge experiments, as expected, the CFU counts in the BCG group were significantly lower than in the naive group in all three organs (p < 0.05, Figure 1). In the first challenge experiment, the CFU counts in all three organs in the DMM group were significantly lower than the naive group (p < 0.05). The CFU counts in the MDM group in all three organs were not significantly different from the naïve control group. However, in the second challenge experiment, the lung was the only organ in which the CFU counts in the DDDM group were significantly lower than the naïve control group (p < 0.05). The liver and spleen counts were not significantly different between these two groups. The DMM/MDM/DDDM group CFU counts were not significantly different from the BCG group in any organ, in either experiment.

These results demonstrate the immunogenicity and protective efficacy against *M*. *tuberculosis* of a MVA and DNA vaccine vectors that induce gamma-interferon secreting CD4 T lymphocyte responses and also of heterologous prime-boost immunisation regimes using these vaccines. DNA vaccination is known to induce a T_{H}1 type immune response, and therefore we chose the quantification of a T_{H}1 cytokine, IFN-γ, in an ELISPOT assay as our functional outcome measure. This assay is a very sensitive method of quantifying T cell function [13]. Proliferation assays are an alternative measure of CD4+ T cell response, but this is not a readout of an effector response and importantly gamma-interferon secretion and proliferation responses are often negatively correlated (Troye-Blomberg et al., Flanagan et al 2000). There are two reasons why measuring IFN-γ production is a more relevant outcome measure in an *M. tuberculosis* challenge model. IFN-γ is an essential component of the protective immune response to tuberculosis, as IFN-γ knockout mice are much more susceptible to challenge with *M tuberculosis* than their wild type counterparts [14]. In addition, a mutation in the human IFN-γ receptor gene confers susceptibility to atypical mycobacterial infection [15].

The recombinant MVA as well as the DNA vaccine each individually generated specific IFN-γ secreting CD4+ T cells to the same peptides. There were no IFN-γ secreting CD8+ T cell responses observed to these constructs, presumably as a results of the absence of a peptide with high binding affinity for the relevant MHC class I molecules in this strain of mice (C57/BL6). As the peptides used to assess the responses spanned the length of both antigens this effectively excludes the presence of a CD8 epitope for this mouse strain. These constructs therefore allowed us to assess the effect of each vaccine type and of prime-boost regimes on CD4+ T cell responses. Although each vaccine type clearly induced CD4 T cell responses heterologous prime-boost regimes with the two vaccines generated stronger CD4+ T cell responses than homologous boosting. Interestingly, the order in which the two vaccines were given made no clear difference to the strength of the immune responses generated. Priming with DNA and boosting with MVA, or priming with MVA and boosting with DNA both produced a 3-4 fold increase in the number of IFN-γ secreting CD4+ T cells specific for the first ESAT 6 peptide. This contrasts with published work on CD8+ T cell responses, where DNA prime followed by MVA boost is the only order in which high levels of immunogenicity and protection are seen [8].

At eight weeks, levels of protection with DNA/MVA immunisation regimes were equivalent to those obtained with BCG and the protection in the BCG immunised group is in the same order as that previously published [6].

In the first challenge experiment, the group immunised with DNA/MVA showed levels of protection equivalent to BCG in all three organs. In the second experiment, protection in the DNA/MVA immunised group was only seen in the lungs at eight weeks. Previous authors have observed varying protective effects in different organs depending on the time from challenge to harvesting. Zhu *et al.* reported protection after DNA immunisation in the lungs four weeks after challenge, but only observed protection in the lungs and spleen 12 weeks after challenge [16]. As the primary route of infection in humans is the pulmonary route, the lung is the most relevant organ in which to identify protection. More relevant aerosol models of M .tuberculosis challenge have been developed, and it will be important to see whether vaccines that confer protection against a systemic route of challenge remain protective against an aerosol challenge, and whether protection in the lungs is maintained.

DNA priming seemed to be necessary for protection to occur in the challenge experiments as in the first challenge experiment, protection was seen in the DMM group but not the MDM group. Note that the lack of protection in the MDM group at 24 hours and at eight weeks effectively rules out a non-specific protective effect of the subunit vaccines administered up to two weeks before challenge. It is uncertain why protection was achieved in the DMM but not the MDM groups when the immunisation order (Table 2) appeared not to affect immunogenicity. The difference however, may relate to the timing of the second MVA boost, as the two MVA doses were given a month apart in the M/D/M group. It may be that within this interval an antibody response to the MVA abrogated the boosting effect.

The mechanism by which the response to a DNA priming vaccination can be boosted by a subsequent immunisation with a recombinant virus encoding the same antigen has not been fully elucidated. Without wishing to be bound by theory, the present inventors predict that it may relate to the induction by DNA of memory T cells to an immunodominant epitope(s), that expand rapidly on exposure to a recombinant virus carrying the same epitope [18]. It is possible that the mechanisms involved in the boosting of CD8+ T cells are different to those involved in the boosting of CD4+ T cells.

### Example 1B - Antigen 85A

DNA and MVA constructs expressing antigen 85A were used to immunise two strains of mice: BALB/c and C57BL/6. Several peptide responses were detected in the splenocytes from immunised mice using the IFN-γ Elispot assay and the overlapping peptides spanning the length of antigen 85A. Mice were immunised with DNA and/or MVA, alone and in combination, in order to determine the optimal immunisation regimens.

All the strongest responses identified were in BALB/c mice. All further work with these constructs was restricted to this mouse strain. The DNA and MVA constructs induced responses to the same peptides. Responses to four of the overlapping peptides were identified using the splenocytes of immunised mice; p11, p15, p24 and p27. The strongest responses were to peptides p 11 and p 15.

### Depletion Studies

To assess the phenotype of the T cell responses to these peptides, CD4+ and CD8+ cells were depleted using magnetic beads. The response to p11 was almost completely abrogated by CD8+ T cell depletion. The response to p15 was completely abrogated by CD4+ T cell depletion. The weaker responses to peptides 24 and p27 were both completely abrogated by CD4+ T cell depletion. These results are summarised in Table 3.

In summary, immunisation with the DNA and MVA constructs expressing antigen 85A induced an immunodominant CD8+ epitope (p11), an immunodominant CD4+ epitope (p15) and 2 weaker CD4+ epitopes (p24 and p27).

**Table 3: Depletion studies on responses identified within antigen 85A.**

| **Peptide** | **Sequence** | **Undepleted response (SFC)** | **CD4⁺ Depletion (SFC)** | **CD8⁺ Depletion (SFC)** |
|---|---|---|---|---|
| P11 | EWYDQSGLSVVMPVGGQSSF | 250 | 200 | 10 |
| P15 | *TFLTSELPGWLQANRHVKPT* | 300 | 0 | 250 |
| P24 | QRNDPLLNVGKLIANNTRVW | 30 | 0 | 20 |
| P27 | LGGNNLPAKFLEGFVRTSNI | 30 | 0 | 25 |

The responses generated by a single immunisation with either construct were weak and only slightly increased by homologous boosting with the same construct. Heterologous boosting of DNA with MVA produced significantly higher frequencies of both the CD4+ (p15) and CD8+ (p11) T cells. Heterologous boosting of MVA with DNA boosted the frequency of CD4+ T cells (p15) but did not increase the CD8+ (p11) T cell response. Three DNA immunisations followed by a single MVA boost (DDDM) generated the highest frequency of IFN-γ secreting T cells. This is consistent with the results using the ESAT6/MPT63-expressing constructs, where the most immunogenic regime was DDDMM.

These results are summarised in table 4.

**Table 4: Summary of peptide specific T cell responses to the antigen 85A constructs.**

| **Immunisation regime** | **SFC per 10⁶ splenocytes to individual peptides^{a} Peptide number** | | | |
|---|---|---|---|---|
| | **11** | **15** | **24** | **27** |
| **DNA x 1** | 29 | 24 | 32 | 19 |
| **DNA x 2** | 11 | 14 | 10 | 16 |
| **DNA x 3** | 70 | 31 | 15 | 16 |
| **MVA x 2** | 9 | 12 | 10 | 6 |
| **DNA/MVA** | 80 | 77 | 9 | 32 |
| **MVA/DNA** | 8 | 103 | 9 | 10 |
| **DNAx3/MVA** | 312 | 325 | 14 | 141 |

| | | | | |
|---|---|---|---|---|
| ^{a}Numbers represent means of SFC per 10⁶ splenocytes for 3-10 mice per group. Standard error is < 20%. | | | | |

### Challenge Experiments

Once optimal immunisation regimes with the antigen 85A constructs had been determined, the protective efficacy of these regimes was evaluated in challenge studies with *M.tb.* Initially, 1 x 10⁶ cfu *M.tb* was used as a challenge dose, half a log lower than the challenge dose used for the C57BL/6 mice. This was because the literature suggests that BALB/c mice are slightly more susceptible to challenge with *M.tb* than C57BL/6 mice. Eight weeks was chosen as a time point for harvest, to enable comparison with the challenge results using the ESAT6/MPT63 constructs. Previous authors have shown a protective effect in BALB/c mice at 6 weeks after an i.p challenge with *M.tb*.

### Comparison of heterologous vs homologous prime-boost regimes

A challenge experiment was set up to compare the protective efficacy of heterologous and homologous prime-boost regimes using the antigen 85A expressing constructs. The immunogenicity results using these constructs had confirmed that heterologous boosting produced higher levels of specific CD4+ and CD8+ T cells than homologous boosting. A control group of mice that received 3 doses of antigen 85A DNA followed by a single dose of non-recombinant MVA were included to assess the specificity of the boosting effect of MVA.

There were 5 groups in this experiment:
- Naïve
- BCG
- DDD
- DDDM
- DDD(Non-recombinant MVA[NRM])

There were 10 mice in all groups except the DDDM group, which had 7 mice. Two to three mice were harvested from each group for immunogenicity at the time of challenge. BCG was given at the same time-point as the first DNA immunisation. Mice were challenged with 10⁶cfu *M.tb* i.p., 2 weeks after the final immunisation, and harvested 8 weeks after challenge.

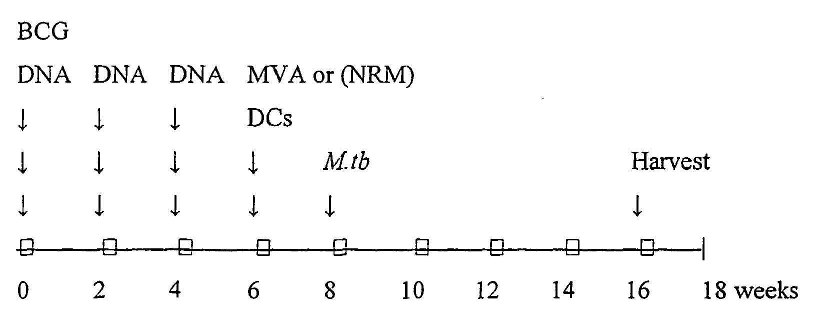

The Elispot results for the DDDM group showed high levels of T cell responses consistent with the previous immunogenicity results for this regime. The results for the DDD and DDD(NRM) groups showed low level responses. There was no boosting effect seen with non-recombinant MVA. These results are summarised in table 5.

**Table 5: Challenge 4: Mean SFC per 10⁶ splenocytes for heterologous and homologous immunisation regimes**

| | P11 | P15 | P24 | P27 |
|---|---|---|---|---|
| DDD | 9 | 0 | 0 | 0 |
| DDD (NRM) | 0 | 8 | 0 | 7 |
| DDDM | 217 | 245 | 0 | 217 |

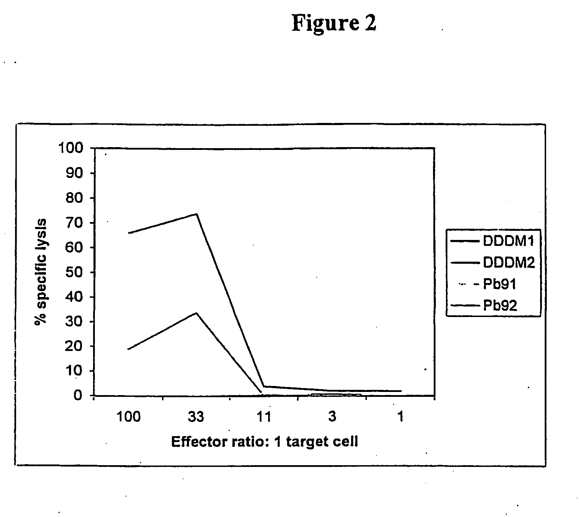

*A ⁵¹ Cr release assay was performed on the splenocytes from mice in the DDDM group. The results of this assay showed that in one of the two mice harvested in the DDDM group, high levels of specific lysis (60-70%) could be demonstrated. In the other mouse, the level of lysis was much lower (20-30%). These results are summarised in* *Figure 2**.*

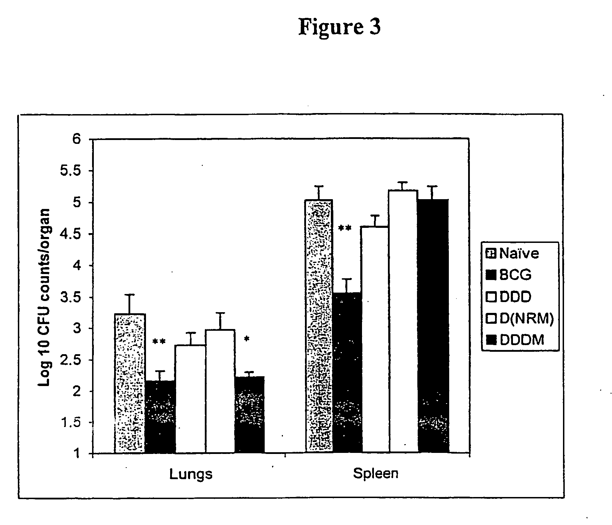

The heterologous prime-boost regime (DDDM) confers protection in the lungs equivalent to BCG when compared to the naïve control group (p=0.010). No protection is seen in the spleen in the DDDM group. The homologous regime, DDD, both alone and boosted with non-recombinant MVA, DDD(NRM), did not confer any significant protection against challenge. As expected, there is a significant protective effect of BCG in both the lungs and spleen, when this group is compared to the naive control group (lungs: p=0.009; spleen: p<0.001). These results are summarised in Figure 3.

### Discussion

The DNA vaccine and recombinant MVA expressing antigen 85A generated specific IFN-y secreting CD4+ and CD8+ T cells to the same four peptides. Heterologous prime-boost regimes with the two vaccines generated higher frequencies of T cell responses than homologous boosting. CD4+ T cell responses were increased regardless of the order of immunisation. This is consistent with the results obtained with the ESAT6/MPT63 expressing constructs (Example 1A). The CD8+ T cell response induced by the antigen 85A expressing constructs was only boosted with the DNA prime-MVA boost immunisation regime and not when the constructs were given in the opposite order. This is consistent with previously published work on boosting CD8+ T cell responses.

The processing pathways for the induction of class-I restricted CD8+ T cell responses and class-II restricted CD4+ T cell responses are different. This may explain why DNA immunisation boosts CD4+, but not CD8+ T cell responses. DNA vaccination is known to be good at priming class-I restricted CD8+ T cells, as the endogenously produced antigen can access the class I pathway. Recombinant viral vectors probably also use the endogenous class I pathway. It may be that the MVA immunisation induces different cytokines to the DNA immunisation. One possible cytokine is IL4. The boosting effect of MVA can be abrogated by the co-administration of IL4 antibodies with the MVA (Sheu, personal communication). IL4 may be necessary to boost a memory CD8+ response but not a CD4+ response.

In the challenge experiment, the enhanced CD4+ and CD8+ T cell responses induced with the heterologous prime-boost immunisation regimes conferred protection in the lungs equivalent to, but not greater than BCG.

### Materials and methods

*M tuberculosis* stocks. *M tuberculosis* (H37Rv) was grown in Dubos medium and incubated at 37°C for 21-28 days. The solution was centrifuged, resuspended in TSB/glycerol and stored at -70°C after titration. Stock solutions were sonicated before use.

Plasmid DNA constructs *M. tuberculosis (H37Rv)* was heat inactivated and DNA extracted (QIAamp,Qiagen, Hilden, Germany). Oligonucleotide primers (Genosys Biotechnologies Ltd, Pampisford, Cambs) were used to amplify the ESAT6 and MPT63 gene. The PCR products were extracted from agarose gel and purified (QIAquick kit, Qiagen). The tissue plasminogen activator (TPA) leader sequence was also amplified. The three PCR products were sequenced, then ligated together to form a single coding sequence with the Pk antibody epitope at the 3' end (TEMPk). The TEMPk fragment was ligated into the plasmid vector pSG2, creating pSG2.TEMPk. This plasmid has the CMV promoter with intron A, the bovine growth hormone poly A sequence, and the kanamycin resistance gene as a selectable marker. Expression of the TEMPk fusion protein in COS-1 cells was detected by immunofluorescence using antibodies to the Pk tag (Serotech, UK) followed by fluoroscein isothiocyanate isomer (FITC) labelled secondary antibodies (Sigma). Nuclear staining showed the protein to be in the cytoplasm. Plasmid DNA for injections was purified using anion exchange chromatography (Qiagen) and diluted in endotoxin free phosphate buffered saline (Sigma).

Construction of Recombinant Modified Vaccinia Ankara (MVA) The DNA sequence TEMPk was cloned into the Vaccinia shuttle vector pSC11. BHK cells were infected with wild type MVA (A Mayr, Veterinary Faculty, University of Munich, Germany) at a multiplicity of infection of 0.05, then transfected with the recombinant shuttle vector. Recombinant virus was selected for with bromodeoxyuridine and then plaque purified on CEF cells.

Animals and immunisations Female C57/BL6 mice aged 4-6 weeks (Harlan Orlac, Shaws Farm, Blackthorn, UK) were injected with plasmid DNA (25µg/muscle) into both tibialis muscles, under anaesthesia. Recombinant MVA (10⁶ pfu) was injected intradermally. Mice were immunised at two week intervals and harvested for immunogenicity two weeks after the last immunisation. For the challenge experiments mice were infected two weeks after the last immunisation. A BCG control group was immunised with 4 x 10⁵cfu *M. bovis BCG* (Glaxo) intradermally at the time of the first DNA/MVA immunisation.

Preparation of splenocytes Mice were sacrificed and spleens removed using aseptic technique. Spleens were crushed and the resulting single cell suspension filtered through a strainer (Falcon, 70µm, Becton Dickson, New Jersey). Cells were pelleted and the red blood cells lysed using a hypotonic lysis buffer. Cells were then washed and counted. Splenocytes were resuspended in alpha-MEM medium with 10%FCS, 2mM glutamine, 50U/ml penicillin, 50 mg/ml streptomycin, 50µM 2-mercaptoethanol and 10mM Hepes pH 7.2 (all from Gibco).

Peptides Overlapping peptides spanning the length of both antigens were purchased from Research Genetics (Huntsville, AL, USA). The peptides were 15 amino acids in length and overlapped by 10 amino acids (Table 1).

ELISPOT assays The number of IFN-γ secreting peptide-specific T cells was determined using the overlapping peptides in an ELISPOT assay [8]. Briefly, 96 well nitro-cellulose plates (Milliscreen MAHA, Millipore, Bedford, MA) were coated with 15µg/ml of the anti-mouse IFN-γ monoclonal antibody R4-6A2 (hybridoma purchased from the European Collection of Animal Cell Cultures). After incubating at 4°C overnight, the wells were washed with PBS and blocked with 100µl RPMI:10% FCS for one hour at room temperature. Splenocytes were added to the wells (10⁶ cells/well) with peptide (final concentration 2µg/ml). Conconavalin A (Sigma-Aldrich Co Ltd, Poole, UK) was used as a positive control for the assay. Control wells had no peptide. After incubating the plate overnight at 37°C with 5% CO₂ in air, it was developed as previously described [8]. The spots were counted using a dissecting microscope. Numbers refer to spot forming cells per million effector cells (SFC).

Cell depletions CD4+ and CD8+ T cell depletions were performed using anti-CD4 or anti-CD8 monoclonal antibodies conjugated to ferrous beads (Dynal, Oslo). Splenocytes from immunised mice were restimulated in six well tissue culture plates with 1 µg/ml of the relevant peptide, and on day 3 of culture 10U/ml of human IL2 (Lymphocult-T, Biotest, Dreieich, Germany) was added. At days 5-7 the restimulated splenocytes were washed twice and incubated on ice for 30 minutes with one of the 2 antibodies (bead:cell = 5:1). An ELISPOT assay was then performed as before, using the depleted cell populations. Assays for peptides E1 and E2 were also performed ex-vivo. Depletion studies for each peptide response were performed twice.

Challenge experiments Mice were infected with 5 x 10⁶ cfu *M.tuberculosis* (H37Rv) by intraperitoneal injection, in a Category III isolator unit. To assess the baseline level of infection, the liver, lungs and spleen from 2-5 mice from each group were harvested and weighed, twenty-four hours after infection. The organs from the remaining 7-10 mice in each group were harvested eight weeks after challenge. Organs were homogenised by vortexing with 5mm glass beads in 1ml of sterile PBS and serial dilutions were plated onto Middlebrook plates. Plates were incubated for 21 days at 37°C and colony counts/gram tissue were then calculated. The Mann-Whitney U test was used to compare CFU counts between groups.

Cell preparation Peripheral blood mononuclear cells (PBMC) were prepared from peripheral blood by Ficoll separation. Assays were either performed on fresh blood, or frozen in 10% DMSO/90% FCS before being assayed, as detailed in the text. All culture medium was supplemented with 10% human AB serum, 2 mM Glutamine and 100 U ml-1 Penicillin/Streptomycin. Cells were depleted using the Dynal Dynabead system at 5-10 beads/cell.

Ex vivo ELISPOT assays. The culture medium was RPMI 1640. ELISPOTs were performed on Millipore MAIP S45 plates with MabTech antibodies according to the manufacturer's instructions: 4 x 105 PBMC were incubated for 18-20 h on the ELISPOT plates in the presence of peptides each at 25 µg ml-1. The plates were then washed in Phosphate Buffered Saline (PBS) containing 0.5% Tween-20 (PBST), and a biotinylated anti-IFNγ antibody diluted in PBS was added, and incubated for 2-24h, the plates were then washed in PBST, and streptavidin alkaline phosphatase diluted 1:1000 in PBS was added. After 1-2 h at room temperature, the plates were washed and developed using the BioRad precipitating substrate kit. Plates were counted by the AutoImmun Diagnostika system. Results are expressed as spot forming units (sfu) per million cells added to the well and are calculated as the difference between the test and the response to medium alone.

### REFERENCES

1 Fine, P.E. and Rodrigues, L.C., Lancet 1990. 335: 1016-1020.
2 Orme, I.M., J.Immunol. 1987. 138: 293-298.
3 Barnes, P.F., Bloch, A.B., Davidson, P.T. and Snider, D.E., Jr., N.Engl.J.Med. 1991. 324: 1644-1650.
4 Flynn, J.L., Goldstein, M.M., Triebold, K.J., Koller, B. and Bloom, B.R., Proc.Natl.Acad.Sci. U.S.A. 1992.89: 12013-12017.
5 Lalvani, A., Brookes, R., Wilkinson, R.J., Malin, A.S., Pathan, A.A., Andersen, P., Dockrell, H., Pasvol, G. and Hill, A.V., Proc.Natl.Acad.Sci. U.S.A. 1998. 95: 270-275.
6 Tascon, R.E., Colston, M.J., Ragno, S., Stavropoulos, E., Gregory, D. and Lowrie, D.B., Nat.Med. 1996. 2: 888-892.
7 Huygen, K., Content, J., Denis, O., Montgomery, D.L., Yawman, A.M., Deck, R.R., DeWitt, C.M., et al, Nat.Med. 1996.2: 893-898.
8 Schneider, J., Gilbert, S.C., Blanchard, T.J., Hanke, T., Robson, K.J., Hannan, C.M., Becker, M., et al, Nat.Med. 1998. 4: 397-402.
9 Sedegah, M., Jones, T.R., Kaur, M., Hedstrom, R., Hobart, P., Tine, J.A. and Hoffman, S.L., Proc.Natl.Acad.Sci. U.S.A. 1998. 95: 7648-7653.
10 Harboe, M., Oettinger, T., Wiker, H.G., Rosenkrands, I. and Andersen, P., Infect.Immun. 1996. 64: 16-22.
11 Brandt, L., Oettinger, T., Holm, A., Andersen, A.B. and Andersen, P., J.Immunol. 1996. 157: 3527-3533.
12 Manca, C., Lyashchenko, K., Wiker, H.G., Usai, D., Colangeli, R. and Gennaro, M.L., Infect.Immun. 1997. 65: 16-23.
13 Lalvani, A., Brookes, R., Hambleton, S., Britton, W.J., Hill, A.V. and McMichael, A.J., J.Exp.Med. 1997. 186: 859-865.
14 Cooper, A.M., Dalton, D.K., Stewart, T.A., Griffin, J.P., Russell, D.G. and Orme, I.M., J.Exp.Med. 1993. 178: 2243-2247.
15 Newport, M.J., Huxley, C.M., Huston, S., Hawrylowicz, C.M., Oostra, B.A., Williamson, R. and Levin, M., N.Engl.J.Med. 199.6: 335:1941-1949.
16 Zhu, X., Venkataprasad, N., Thangaraj, H.S., Hill, M., Singh, M., Ivanyi, J. and Vordermeier, H.M., J.Immunol. 1997. 158: 5921-5926.
17 Tighe, H., Corr, M., Roman, M. and Raz, E., Immunol.Today 1998.19: 89-97.
18 Schneider, J., Gilbert, S.C., Hannan, C.M., Degano, P., Prieur, E., Sheu, E.G., Plebanski, M. and Hill, A.V.S., Imnunological Reviews 1999. 170: 29-38.
19 Blanchard, T.J., Alcami, A., Andrea, P. and Smith, G.L., J.Gen. Virol. 1998. 79: 1159-1167.
20 Rosenberg, E.S., Billingsley, J.M., Caliendo, A.M., Boswell, S.L., Sax, P.E., Kalams, S.A. and Walker, B.D., Science 1997. 278: 1447-1450.
21 Stoute, J.A., Slaoui, M., Heppner, D.G., Momin, P., Kester, K.E., Desmons, P., Wellde, B.T., et al, N.Engl.J.Med. 1997. 336: 86-91.
22 Lalvani, A., Moris, P., Voss, G., Pathan, A.A., Kester, K.E., Brookes, R., Lee, E., et al, J.Infect.Dis. 1999. 180: 1656-1664.
23 Gilbert SC, Plebanski M, Harris SJ, Allsopp CE, Thomas R, Layton GT, Hill AV. Nat Biotechnol. 1997 Nov;15(12):1280-4.
24. Robson KJ, Hall JR, Jennings MW, Harris TJ, Marsh K, Newbold CI, Tate VE, Weatherall DJ. Nature. 1988 Sep 1;335:79-82.

## Claims

1. Use of
(a) a first composition comprising a source of one or more CD4+ T cell epitopes of a tuberculosis antigen; and
(b) a second composition comprising a source of one or more CD4+ T cell epitopes of the tuberculosis antigen, including at least one CD4+ T cell epitope which is the same as a CD4+ T cell epitope of the first composition,
wherein the source of T cell epitopes in the first composition is not a poxvirus vector,
wherein the source of CD4+ epitopes for the second composition is a non-replicating or replication impaired recombinant poxvirus vector;
for the manufacture of a medicament for inducing a T_{H}1-type CD4+ T cell response against the tuberculosis antigen, wherein the first and second compositions are for administration in either order.

2. Use according to claim 1, wherein the vector is of the modified vaccinia virus Ankara strain or derivative thereof.

3. Use according to claim 1, wherein the vector is a fowlpox vector or derivative thereof.

4. Use according to any of the preceding claims, wherein the epitopes in or encoded by the first or second composition are provided in a sequence which does not occur naturally as the expressed product of a gene in *M tuberculosis.*

5. Use according to any of the preceding claims, wherein at least one dose of a composition is for administration by an intradermal route.

6. Use according to any of the preceding claims, for administration of at least one dose of the first composition followed by at least one dose of the second composition.

7. Use according to claim 6, for administration of a plurality of sequential doses of the first composition followed by at least one dose of the second composition.

8. Use according to claim 7, for administration of three sequential doses of the first composition followed by one dose of the second composition.

9. Use of a source of one or more CD4+ T cell epitopes of a tuberculosis antigen, wherein the source of CD4+ epitopes is a non-replicating or replication impaired recombinant poxvirus vector, for the manufacture of a medicament for boosting a primed TH₁-type CD4+ T cell response against the tuberculosis antigen.

10. Use according to any of claims 1 to 3 or 5 to 9, wherein the tuberculosis antigen is ESAT6, MPT63 or 85A.

## Patentansprüche

1. Verwendung
(a) einer ersten Zusammensetzung, die eine Quelle eines oder mehrerer CD4⁺-T-Zell-Epitope eines Tuberkuloseantigens aufweist; und
(b) einer zweiten Zusammensetzung, die eine Quelle eines oder mehrerer CD4⁺-T-Zell-Epitope des Tuberkuloseantigens, einschließlich zumindest ein CD4⁺-T-Zell-Epitop, das das gleiche CD4⁺-T-Zell-Epitop wie das der ersten Zusammensetzung ist, aufweist,
wobei die Quelle der T-Zell-Epitope in der ersten Zusammensetzung kein Pockenvirusvektor ist,
wobei die Quelle der CD4⁺-Epitope für die zweite Zusammensetzung ein nichtreplizierender oder ein replikationsgeschädigter rekombinanter Pockenvirusvektor ist;
zur Herstellung eines Arzneimittels für die Induktion einer T_{H}1-Typ-CD4⁺-T-Zell-Antwort gegen das Tuberkuloseantigen, wobei die erste und zweite Zusammensetzung für eine Verabreichung in beliebiger Reihenfolge ausgestaltet sind.

2. Verwendung gemäß Anspruch 1, wobei der Vektor von einem modifizierten Vacciniavirus des Stammes Ankara oder Abkömmlingen hiervon stammt.

3. Verwendung gemäß Anspruch 1, wobei der Vektor ein Geflügelpockenvektor oder ein Abkömmling hiervon ist.

4. Verwendung gemäß einem der vorherigen Ansprüche, wobei die Epitope in oder kodiert von der ersten oder zweiten Zusammensetzung in einer Sequenz bereitgestellt werden, die nicht natürlich als exprimiertes Produkt eines Genes in M. *tuberculosis* vorkommt.

5. Verwendung gemäß einem der vorherigen Ansprüche, wobei zumindest eine Dosis einer Zusammensetzung für die Verabreichung über einen intradermalen Weg bereitgestellt wird.

6. Verwendung gemäß einem der vorherigen Ansprüche, zur Verabreichung von zumindest einer Dosis der ersten Zusammensetzung gefolgt von zumindest einer Dosis der zweiten Zusammensetzung.

7. Verwendung gemäß Anspruch 6, zur Verabreichung einer Vielzahl von aufeinanderfolgenden Dosen der ersten Zusammensetzung gefolgt von zumindest einer Dosis der zweiten Zusammensetzung.

8. Verwendung gemäß Anspruch 7, zur Verabreichung von drei aufeinanderfolgenden Dosen der ersten Zusammensetzung gefolgt von einer Dosis der zweiten Zusammensetzung.

9. Verwendung einer Quelle von einem oder mehreren CD4⁺-T-Zell-Epitopen eines Tuberkuloseantigens, wobei die Quelle der CD4⁺-Epitope ein nichtreplizierender oder replikationsgeschädigter rekombinanter Pockenvirusvektor ist, zur Herstellung eines Arzneimittels zur Verstärkung einer vorbereiteten T_{H}1-Typ-CD4⁺-T-Zellantwort gegen das Tuberkuloseantigen.

10. Verwendung gemäß einem der Ansprüche 1 bis 3 oder 5 bis 9, wobei das Tuberkuloseantigen ESAT6, MPT63 oder 85A ist.

## Revendications

1. Utilisation d'
(a) une première composition comprenant une source d'un ou de plusieurs épitopes de cellule T CD4+ d'un antigène tuberculeux ; et
(b) une seconde composition comprenant une source d'un ou de plusieurs épitopes de cellules T CD4+ de l'antigène tuberculeux, comprenant au moins un épitope de cellule T CD4+ qui est identique à un épitope de cellule T CD4+ de la première composition,
dans laquelle la source d'épitopes de cellule T dans la première composition n'est pas un vecteur poxvirus,
dans laquelle la source d'épitopes CD4+ pour la seconde composition est un vecteur poxvirus recombinant incapable de se répliquer ou présentant une réplication altérée ;
pour la fabrication d'un médicament destiné à induire une réponse de cellule T CD4+ de type TH₁ contre l'antigène tuberculeux, dans laquelle la première et la seconde compositions sont administrées dans n'importe quel ordre.

2. Utilisation selon la revendication 1, dans laquelle le vecteur est la souche Ankara du virus de la vaccine modifié ou un dérivé de celle-ci.

3. Utilisation selon la revendication 1, dans laquelle le vecteur est un vecteur de la variole aviaire ou un dérivé de celui-ci.

4. Utilisation selon l'une quelconque des revendications précédentes, dans laquelle les épitopes dans ou codés par la première ou seconde composition sont fournis dans une séquence qui ne survient pas à l'état naturel sous forme du produit exprimé d'un gène dans *M. tuberculosis.*

5. Utilisation selon l'une quelconque des revendications précédentes, dans laquelle au moins une dose d'une composition est destinée à une administration par une voie intradermique.

6. Utilisation selon l'une quelconque des revendications précédentes, pour l'administration d'au moins une dose de la première composition suivie d'au moins une dose de la seconde composition.

7. Utilisation selon la revendication 6, pour une administration d'une pluralité de doses séquentielles de la première composition suivie d'au moins une dose de la seconde composition.

8. Utilisation selon la revendication 7, pour une administration de trois doses séquentielles de la première composition suivie d'une dose de la seconde composition.

9. Utilisation d'une source d'un ou de plusieurs épitopes de cellule T CD4+ d'un antigène tuberculeux, dans laquelle la source d'épitopes CD4+ est un vecteur poxvirus recombinant incapable de se répliquer ou présentant une réplication altérée, pour la fabrication d'un médicament destiné à stimuler une réponse de cellule T CD4+ de type TH₁ contre l'antigène tuberculeux.

10. Utilisation selon l'une quelconque des revendications 1 à 3 ou 5 à 9, dans laquelle l'antigène tuberculeux est ESAT6, MPT63 ou 85A.
